# EUROPEAN PATENT APPLICATION

(11) **EP 3 656 370 A1**
(43) Date of publication of application: **27.05.2020**
(21) Application number: 18207558.0
(22) Date of filing: 21.11.2018
(51) Int. Cl.: A61K 8/66, A61Q 11/00, A61K 8/9789

(54) **PHARMACEUTICAL COMPOSITION AND ITS USE FOR THE PREVENTION AND TREATMENT OF ORAL CAVITY PATHOLOGIES**

(71) Applicant: Grossi, Lionello, 20010 Pregnana MI (IT)
(72) Inventor: Grossi, Lionello, 20010 Pregnana MI (IT)
(74) Representative: Biggi, Cristina

(57) **Abstract**

The invention relates to a pharmaceutical composition comprising lysozyme, dextranase and an essential oil of a plant belonging to the family Myrtaceae for the prevention, treatment and follow-up of pathologies of the oral cavity of infectious, inflammatory, degenerative or autoimmune origin, preferably selected in the group consisting of: gingivitis, periodontitis, aphthous stomatitis, herpetic stomatitis, prosthetic stomatitis, recurrent aphthosis, xerostomia, xerostomia due to Sjögren's syndrome, burning mouth syndrome (BMS), oral candidiasis, inflammatory dermatoses such as oral lichen planus, autoimmune disorders of the mucous membranes such as mucous membrane pemphigoid (MMP), graft-versus-host disease, white tongue, migratory glossitis, desquamative gingivitis due to oral lichen planus, herpes and combinations thereof.

The composition is preferably formulated, together with other ingredients, as a mouthwash for oral rinses.

## Description

### TECHNICAL FIELD

The present invention relates to a pharmaceutical composition and the use thereof for the prevention, treatment and follow-up of pathologies of the oral cavity.

### BACKGROUND OF THE INVENTION

There are known compositions, in particular formulated as mouthwashes, for washing the oral cavity with the aim of preventing or treating infections and the excess bacterial biofilm that forms inside the mouth. Mouthwashes are also used for analgesic, anti-inflammatory, antibacterial or antifungal purposes to prevent or treat pathologies that cause pain, infection, inflammation or other symptoms. Furthermore, mouthwashes are generally used for cosmetic purposes to control bad breath and unpleasant odours and help prevent the formation of cavities.

Over time numerous mouthwash formulations have been developed which contain different types of ingredients depending on the cosmetic or pharmaceutical action it is desired to obtain. For example, there are known mouthwashes containing essential oils, fluoride-based compounds, antiseptic compounds, anti-inflammatory drugs and plant extracts. Chlorhexidine is an antiseptic compound (disinfectant and antibacterial) that is among the most used in the formulation of mouthwashes for the prevention and treatment of gum and oral cavity disorders. More precisely, mouthwash with chlorhexidine is effective in controlling bacterial plaque, a sticky coating consisting of millions of bacteria immersed in a matrix that adheres like glue to the tooth surface.

In fact, chlorhexidine is a powerful synthetic antibacterial agent with a dual action: bactericidal (it kills germs) and bacteriostatic (it prevents bacterial replication). In mouthwashes the active ingredient is generally chlorhexidine gluconate (salified gluconic acid with chlorhexidine in an aqueous solution).

However, chlorhexidine-based mouthwashes have side effects due to their use for long periods, as they can alter the natural colouring of tooth enamel, causing unattractive stains on teeth (removable exclusively by means of professional dental cleaning).

Other active ingredients with antibacterial and antifungal activity commonly used in mouthwashes are triclosan, benzalkonium chloride, parabens, sodium lauryl phosphate, fluorides and alcohols. These active ingredients too can bring undesirable effects due to their repeated long-term use, such as, for example
- Difficulty in passing through the biofilm
- Imbalance of the oral ecosystem
- Sensitisation of the mucous membranes
- Allergies
- Burning and dryness
- Temporary alteration in taste.

Therefore, in this field there remains a need to provide a pharmaceutical composition applicable to the oral cavity and gums that is effective in the prevention, treatment and follow-up of pathologies without having side effects due to prolonged use.

The pharmaceutical composition must therefore not comprise chlorhexidine triclosan, benzalkonium chloride, parabens, alcohols, sodium lauryl phosphate and/or fluoride as the active ingredients.

### SUMMARY OF THE INVENTION

The present invention relates to a pharmaceutical composition comprising lysozyme, dextranase and an essential oil of a plant belonging to the family Myrtaceae.

The pharmaceutical composition can comprise further excipient substances selected in the group consisting of: papain, carrageenan (Chondrus Crispus), idebenone, extract of Centella asiatica, zeolite, bentonite, kaolin, ionic silver, colloidal silver, colloidal silica, colloidal copper, colloidal gold, casein phosphopeptide, amorphous calcium fluoride, glucose oxidase (GOX), lactoperoxidase, lactoferrin and mixtures thereof.

The composition can be formulated as a mouthwash, solution, suspension, gel, powder to be reconstituted in water, emulsion, oleolite, paste, foam, ointment, poultice, unguent, cream, lozenge, pill, capsule, tablet, or chewing gum.

The invention also relates to the use of the composition for the prevention, treatment and follow-up of pathologies of the oral cavity of infectious, inflammatory, degenerative or autoimmune origin, preferably selected in the group consisting of: gingivitis, periodontitis, aphthous stomatitis, herpetic stomatitis, prosthetic stomatitis, recurrent aphthosis, xerostomia, xerostomia due to *Sjögren's* syndrome, burning mouth syndrome (BMS), oral candidiasis, inflammatory dermatoses such as oral lichen planus, autoimmune disorders of the mucous membranes such as mucous membrane pemphigoid (MMP), graft-versus-host disease, white tongue, migratory glossitis, desquamative gingivitis due to oral lichen planus, herpes and combinations thereof.

### DETAILED DESCRIPTION OF THE INVENTION

The present invention relates to a pharmaceutical composition comprising the active ingredients lysozyme, dextranase and an essential oil of a plant belonging to the family Myrtaceae.

Lysozyme (peptidoglycan N-acetylmuramoyl-hydrolase) is an enzyme naturally present in the saliva of human beings and in other human secretions such as tears. Lysozyme is endowed with bactericidal activity and is capable of damaging the cell wall of several bacteria (Gram+), catalysing the hydrolysis of the beta 1,4 bond between N-acetylmuramic acid (NAM) and N-acetyl glucosamine (NAG), which are the principal component of peptidoglycan. In this manner, lysozyme reduces the negative surface charge of the bacterial membrane and facilitates phagocytosis of the bacteria by the immune system.

Lysozyme is present in the composition of the invention in an amount ranging from 0.03% to 2% by weight, preferably from 0.1% to 0.5% by weight, more preferably from 0.2% to 0.4% by weight.

Dextran is a sticky structure that bacteria form and use to isolate and protect themselves from the external environment, while attracting other pathogenic bacterial species and thereby increasing the volume of plaque and making it even more harmful. In the oral cavity, dietary sugars are converted into dextran by fermenting bacteria: *Streptococcus Mutans* and *Lactobacillus Rhamnosus,* associated with cavity formation.

Dextranase (also called dextrin dextranase) is an enzyme belonging to the class of transferases which is capable of splitting dextran into smaller molecules that cannot be used by bacteria, thus preventing the formation of bacterial biofilm.

Dextranase is present in the composition of the invention in an amount comprised from 0.03% to 2% by weight, preferably from 0.05% to 0.3% by weight, more preferably from 0.05% to 0.1% by weight.

The essential oil of a plant belonging to the family Myrtaceae is selected from: an essential oil of Melaleuca alternifolia (also called tea tree oil), essential oil of Kunzea ericoides (also called kanuka) and essential oil of leptospermum scoparium (also called manuka). The essential oil of a plant belonging to the family Myrtaceae is preferably the essential oil of Melaleuca alternifolia (tea tree oil).

The essential oil is present in the composition in an amount comprised from 0.03% to 3% by weight, preferably from 0.3 to 0.8% by weight, more preferably from 0.4% to 0.6% by weight.

The essential oil of a plant belonging to the family Myrtaceae possesses antibacterial, antimycotic, antiviral and anti-inflammatory activity, but at the same time it also has properties of soothing the oral cavity.

Lysozyme, dextranase and the essential oil of a plant belonging to the family Myrtaceae are substances that are known individually for their antibacterial activity. However, the Applicant has found that the simultaneous application of the three substances leads to a synergistic effect (demonstrated by the experiments included herein), which results in a surprising pharmacological activity against pathologies of the oral cavity (in particular pathologies of the mucosa, teeth and gums) of an infectious, inflammatory, degenerative or autoimmune origin, preferably selected from: gingivitis, periodontitis, aphthous stomatitis, prosthetic stomatitis, recurrent aphthosis, xerostomia, xerostomia due to Sjögren's syndrome, burning mouth syndrome (BMS), oral candidiasis, inflammatory dermatoses such as oral lichen planus, autoimmune disorders of the mucous membranes such as the mucous membrane pemphigoid (MMP), graft-versus-host disease, white tongue, migratory glossitis, desquamative gingivitis due to oral lichen planus, herpes and combinations thereof.

The pharmacological activity of the composition of the invention relates to the prevention, the treatment and the follow-up of one or more pathologies of the oral cavity of an infectious, inflammatory, degenerative or autoimmune origin.

The invention thus relates to a pharmaceutical composition comprising lysozyme, dextranase and an essential oil of a plant belonging to the family Myrtaceae for use as a medication.

The invention also relates to a pharmaceutical composition comprising lysozyme, dextranase and an essential oil of a plant belonging to the family Myrtaceae for use in the prevention, treatment or follow-up of a pathology of infectious, inflammatory, degenerative or autoimmune origin, preferably selected from: gingivitis, periodontitis, aphthous stomatitis, prosthetic stomatitis, recurrent aphthosis, xerostomia, xerostomia due to Sjögren's syndrome, burning mouth syndrome (BMS), oral candidiasis, inflammatory dermatoses such as oral lichen planus, autoimmune disorders of the mucous membranes such as mucous membrane pemphigoid (MMP), graft-versus-host disease, white tongue, migratory glossitis, desquamative gingivitis due to oral lichen planus, herpes and combinations thereof.

In one embodiment, the invention relates to a method for the treatment of a patient affected by one or more pathologies of infectious, inflammatory, degenerative or autoimmune origin, preferably selected in the group consisting of: gingivitis, periodontitis, aphthous stomatitis, prosthetic stomatitis, recurrent aphthosis, xerostomia, xerostomia due to Sjögren's syndrome, burning mouth syndrome (BMS), oral candidiasis, inflammatory dermatoses such as oral lichen planus, autoimmune disorders of the mucous membranes such as mucous membrane pemphigoid (MMP), graft-versus-host disease, white tongue, migratory glossitis, desquamative gingivitis due to oral lichen planus, herpes and combinations thereof, which comprises the administration of an effective dose of the composition of the invention to the patient.

Oral lichen planus is a relatively common inflammatory *mucocutaneous* disease, which is chronic in character and of unknown aetiology. In the inflammatory stage, lichen is defined as a precancerous condition. It is characterised by patches of stripe-like white lines or white spots that mostly appear on the inside of the cheeks.

It can result in the appearance of painful ulcers, bad breath and discomfort during drinking and eating.

Benign mucous membrane pemphigoid manifests itself with mucosal erosions of varying severity, from small and asymptomatic ones to extensive, ulcerous and extremely painful ones with the presence of bad breath. The oral cavity lesions are often whitish and prone to bleeding, either spontaneous or following slight injuries. It is a chronic-recurring pathology.

The conventional therapy presently applied for both pathologies is the administration of cortisone-based and immunosuppressant drugs. The prognosis is normally a poor response to the treatments and rare spontaneous remission.

Desquamative gingivitis due to lichen planus shows a clinical picture characterised by erythema, erosion, blisters and desquamation of the free and attached gingiva. It can manifest itself in very different ways, from symptomless forms with slight modifications of the normal state of the gums to severe clinical conditions with widespread blisters and erosions and severe functional limitation.

The conventional therapy is carried out with the administration of cortisone-based drugs. If it is caused by the use of drugs, they need to be suspended. In some cases it is impossible to interrupt the pharmacological therapies and the patient is forced to continue using cortisone.

Migratory glossitis is a chronic or recurrent benign inflammatory disease of the dorsum of the tongue. The papillae disappear and grow back continually; the most affected areas are the side edges and tip of the tongue. Also known as "geographic tongue" because of the particular form the tongue takes on as a result of the various red patches surrounded by a white edge.

The conventional therapy provides for the administration of cortisone-based drugs or local anaesthetics. The prognosis is normally a foreseeable remission in 2 months after therapy.

Xerostomia results in a dry mouth due to a lack of saliva and causes difficulty in speaking and eating and in relational life. It results in bad breath and an increase in cavities and renders the mucosa of periodontal tissue and of the mouth more vulnerable to infections.

The conventional therapy provides for the administration of cortisone-based drugs, artificial saliva and cholinergic antagonists. The pathology is chronic.

Xerostomia due to Sjögren's syndrome is characterised by a dysfunction of the exocrine glands due to lymphocytic infiltration. It results in a reduction in the secretion of saliva. Therefore, the mucosa of the mouth is dry. It also changes the consistency of salivary fluid: it becomes viscous and dense and contains less lysozyme. In addition to mouth dryness, the disease also manifests itself with difficulty in chewing and swallowing, digestive problems, an increase in the incidence of cavities and periodontitis, oral lesions and bad breath.

The conventional therapy provides for the administration of cortisone-based drugs, artificial saliva and cholinergic antagonists. The pathology is chronic and systemic.

Burning mouth syndrome is a pathological condition in which intense pain, similar to that caused by a burn, is experienced in the oral cavity. The affected parts can be the tongue, lips, gums, cheeks, palate or the entire mouth.

The conventional therapy provides for the administration of antidepressants, artificial saliva, cholinergic antagonists and psychotherapy. The prognosis is generally variable healing with relapses. Graft-versus-host disease (GvHD): it is a medical complication that follows a transplant from a genetically different person. The immune cells (white blood cells) in the donated tissue recognise the receiver as foreign. The transplanted immune cells thus attack the cells of the host's body.

The conventional therapy provides for the administration of steroids and anaesthetics. The prognosis is healing of the plaques in about 20 days. There is no definitive cure as it is a recurrent disease.

Stomatitis is a painful acute inflammation of the mucosa of the mouth which affects the gums, the inside of the cheeks and lips, palate and tongue. It manifests itself with an evident, extensive reddening of the mucosa of the mouth and ulcerous lesions and the appearance of painful aphthae, small blisters that break, giving rise to small greyish-white ulcerations of a bacterial or viral nature, with the presence of bad breath. The conventional therapy provides for the administration of anti-inflammatory drugs with a prognosis of healing after months of therapy. Candidiasis is a mycosis affecting the oral mucosa. Subjectively, there is a burning sensation and often difficulty in eating. The conventional therapy provides for the administration of antimycotic drugs with a prognosis of healing after months of therapy.

Recurrent aphthosis is an inflammatory pathology characterised by recurrent ulcerations of the oral mucosa. It is one of the most frequent pathologies occurring in the oral cavity and affects about 15% of the population. The most aggressive and deepest aphthae (major *aphthous* ulcers) heal in 3 months, leaving scars in their wake.

The conventional therapy provides for the administration of steroids and anaesthetics. The prognosis is remission of the plaques in 20 days. There is no definitive cure, however, as it is a recurrent disease.

All of these pathologies are thus characterised by serious problems as regards the quality of life of the patient, who in the majority of cases manifests pain, discomfort, difficulty in eating and speaking and difficulty in his or her relational life.

The composition of the invention has demonstrated effectiveness in treating all these pathologies already after 15 days or 30 days of treatment, as demonstrated by the clinical tests reported below in the present patent application. This surprising effectiveness displayed by the composition of the invention is ascribable to the synergistic effect of the three active ingredients: dextranase, lysozyme and essential oil of a plant belonging to the family Myrtaceae, as shown by the comparative test results reported in tables 1-3 for the pathologies gingivitis, periodontitis, aphthous stomatitis and prosthetic stomatitis.

The composition of the invention can comprise at least one further ingredient selected from: papain, carrageenan (Chondrus Crispus), idebenone, extract of Centella asiatica, zeolite, bentonite, kaolin, ionic silver, colloidal silver, colloidal silica, colloidal copper, colloidal gold, casein phosphopeptide, amorphous calcium fluoride, glucose oxidase (GOX), lactoperoxidase, lactoferrin and mixtures thereof.

Each of said ingredients, if present in the composition, is in an amount ranging from 0.01 % to 20% by weight, preferably from 0.01% to 10% by weight, more preferably from 0.05% to 5%, even more preferably from 0.05% to 2% by weight.

Other ingredients that can be present in the composition of the invention are a food preservative, for example sodium benzoate, and a food sweetener, for example acesulfame potassium.

The composition is preferably formulated, optionally together with one or more excipients, preservatives and/or sweeteners, as a mouthwash for oral rinses.

Alternatively, the composition can be formulated, optionally together with one or more excipients, preservatives and/or sweeteners, as a solution, suspension, gel, powder to be reconstituted in water, emulsion, oleolite, paste, foam, ointment, poultice, unguent, cream, lozenge, pill, capsule, tablet or chewing gum.

As a further ingredient, the composition of the invention comprises water in the amount necessary to formulate a mouthwash, an aqueous solution, a suspension, a gel, an emulsion, a paste, a foam, an ointment, a poultice or a cream.

The composition does not comprise substances that may provide unwanted effects in the case of repeated use, such as, for example: chlorhexidine and triclosan, benzalkonium chloride, parabens, fluoride, sodium lauryl phosphate, and/or alcohol.

In one embodiment the composition comprises:
- lysozyme in an amount of 0.03% to 2% by weight, preferably 0.1% to 0.5% by weight, more preferably 0.2% to 0.4% by weight;
- dextranase in an amount comprised from 0.03% to 2% by weight, preferably from 0.05% to 0.3% by weight, more preferably from 0.05% to 0.1% by weight;
- essential oil of a plant belonging to the family Myrtaceae in an amount of 0.03% to 3% by weight, preferably 0.3 to 0.8% by weight, more preferably 0.4% to 0.6% by weight;

- one or more further ingredients listed above in an amount of 0.01% to 2%;
- water quantum sufficit.

Said composition is preferably formulated as a mouthwash. The further ingredients are preferably papain, idebenone, extract of Centella asiatica, carrageenan (Chondrus Crispus) and xylitol.

The essential oil of a plant belonging to the family Myrtaceae is preferably essential oil of Melaleuca alternifolia (tea tree oil).

In one embodiment the composition also comprises sodium benzoate as a preservative and acesulfame potassium as a sweetener.

For the treatment of the pathologies indicated, the composition of the invention must be taken at least twice a day by rinsing the mouth in the case of a liquid formulation (mouthwash, solution, suspension) or by application if formulated in a solid form (paste, gel, emulsion, foam, ointment etc.).

The composition of the invention is prepared with conventional methods used for cosmetic and pharmaceutical formulations.

### EXAMPLE 1 - Formulation

### Mouthwash

| | |
|---|---|
| Tea Tree oil | 0.5 % by weight |
| Lysozyme | 0.3 % by weight |
| Dextranase | 0.05 % by weight |

Papain
Xylitol
Preservative: Sodium benzoate
Sweetener: Acesulfame potassium

### EXAMPLE 2 - Formulation

### Mouthwash

| | |
|---|---|
| Tea Tree oil | 0.5 % by weight |
| Lysozyme | 0.3 % by weight |
| Dextranase | 0.05 % by weight |

Idebenone complexed in cyclodextrins
Extract of Centella asiatica
Carrageenan (Chondrus Crispus)
Xylitol
Preservative: Sodium benzoate
Sweetener: Acesulfame potassium

### EXAMPLE 3 - Pharmacological activity

Forty patients aged 30 to 70 years with at least one of the following four pathologies were recruited: gingivitis, periodontitis, aphthous stomatitis and prosthetic stomatitis.
- 3 experimental groups were organised, each made up of 10 people (GROUP 1, GROUP 2, GROUP 3), to whom the mouthwashes described below were administered.
- Finally, a control group was organised, made up of 10 people who followed "traditional" therapies with the use of mouthwashes based on 0.20% chlorhexidine.

The experimental subjects in all 4 groups were selected so that there were:
- 5 people with gingivitis, code 1 or 2 of the PSR*
- 5 people with periodontitis, code 3 or 4 of the PSR*

### *PSR - Periodontal screening and recording

| | |
|---|---|
| Code 0 | Healthy state of the mouth |
| Code 1 | Bleeding on probing |
| Code 2 | Bleeding on probing and tartar |
| Code 3 | Gingival pockets between 3.5 and 5.5 mm |
| Code 4 | Gingival pockets deeper than 5.5 mm |

**RESEARCH DESIGN - Table 1**

| | **TIME 0** | **TIME 1 -1 month-** | **PAUSE 1 -1 month-** | **TIME2 -1 month-** | **PAUSE 2 -1 month-** | **TIME 3 -1 month-** | **TIME 4** |
|---|---|---|---|---|---|---|---|
| **GROUP 1** 5 gingivitis 5 periodontitis | PRELIMINARY EXAMINATION | TEA TREE OIL 0.5% | NO TREATMENT | TEA TREE OIL 0.5% LYSOZYME 0.5% | NO TREATMENT | TEA TREE OIL 0.03% DEXTRANASE 0.03% LYSOZYME 0.03% | FINAL ASSES SMENT |
| **GROUP 2** 5 gingivitis 5 periodontitis | PRELIMINARY EXAMINATION | DEXTRANASE 0.5% | NO TREATMENT | TEA TREE OIL 0.5% DEXTRANASE 0.5% | NO TREATMENT | TEA TREE OIL 1% DEXTRANASE 1% LYSOZYME 1% | FINAL ASSES SMENT |
| **GROUP 3** 5 gingivitis 5 periodontitis | PRELIMINARY EXAMINATION | LYSOZYME 0.5% | NO TREATMENT | DEXTRANASE 0.5% LYSOZYME 0.5% | NO TREATMENT | TEA TREE OIL 0.5% DEXTRANASE 0.05% LYSOZYME 0.3% | FINAL ASSES SMENT |
| **CONTROL GROUP** 5 gingivitis 5 periodontitis | PRELIMINARY EXAMINATION | CHLORHEXIDINE 0.20% | NO TREATMENT | CHLORHEXIDINE 0.20% | NO TREATMENT | CHLORHEXIDINE 0.20% | FINAL ASSES SMENT |

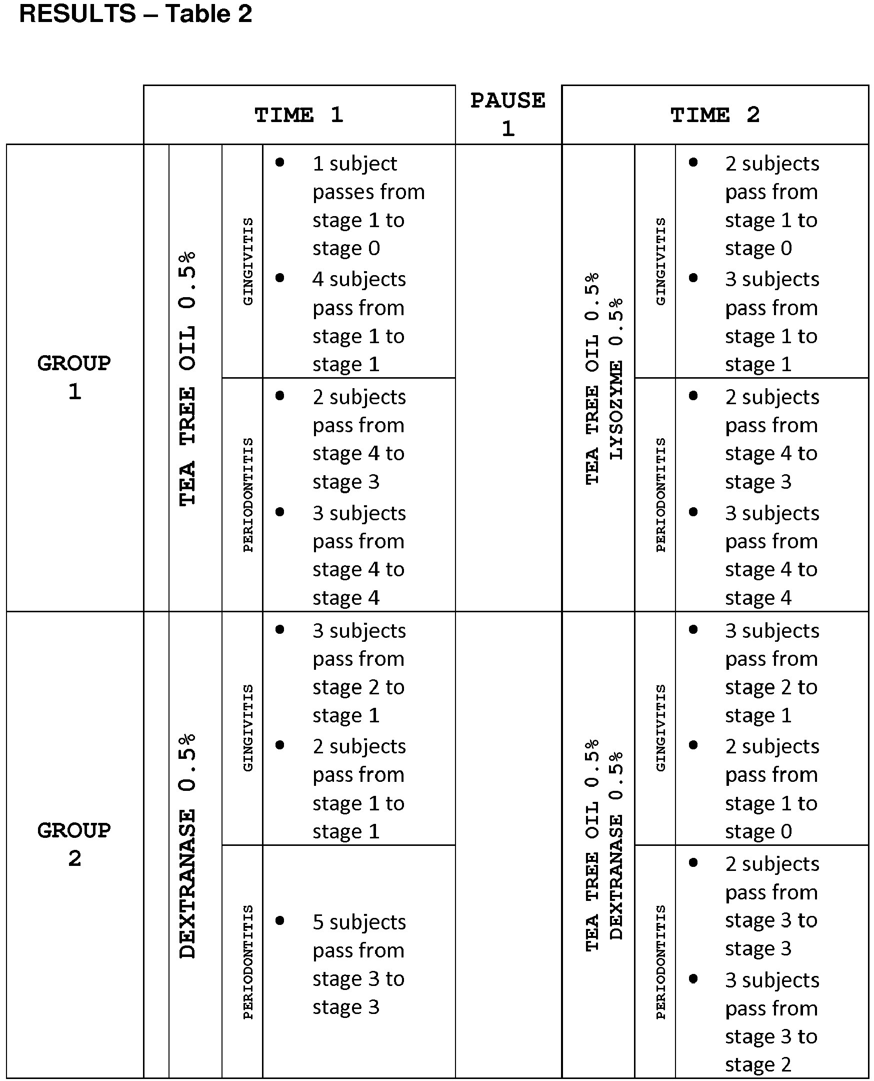

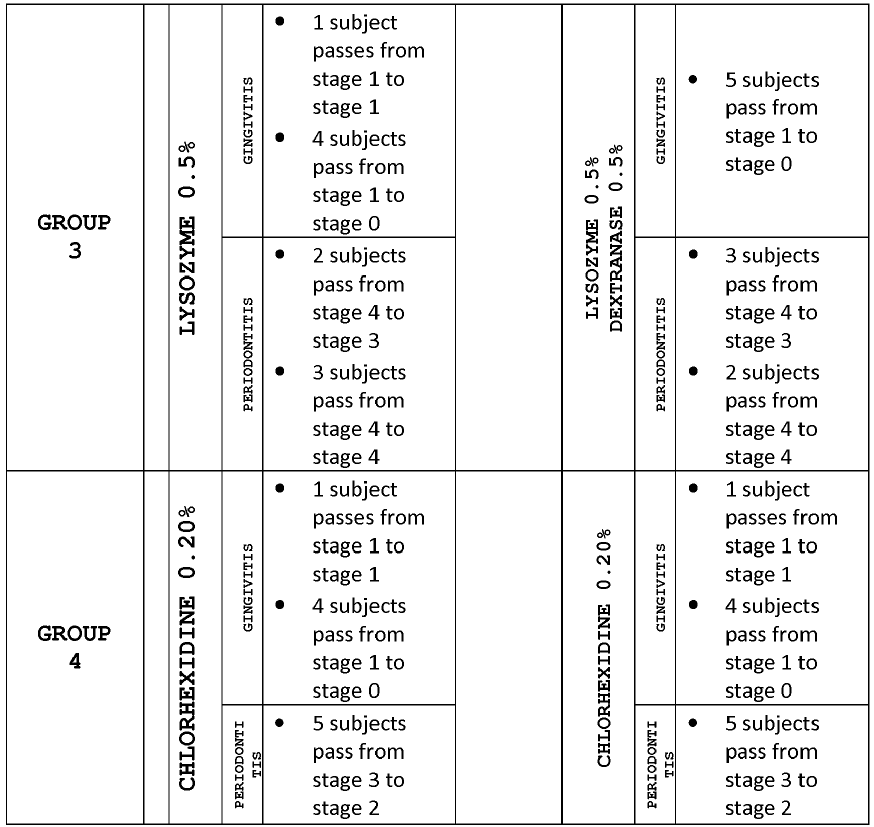

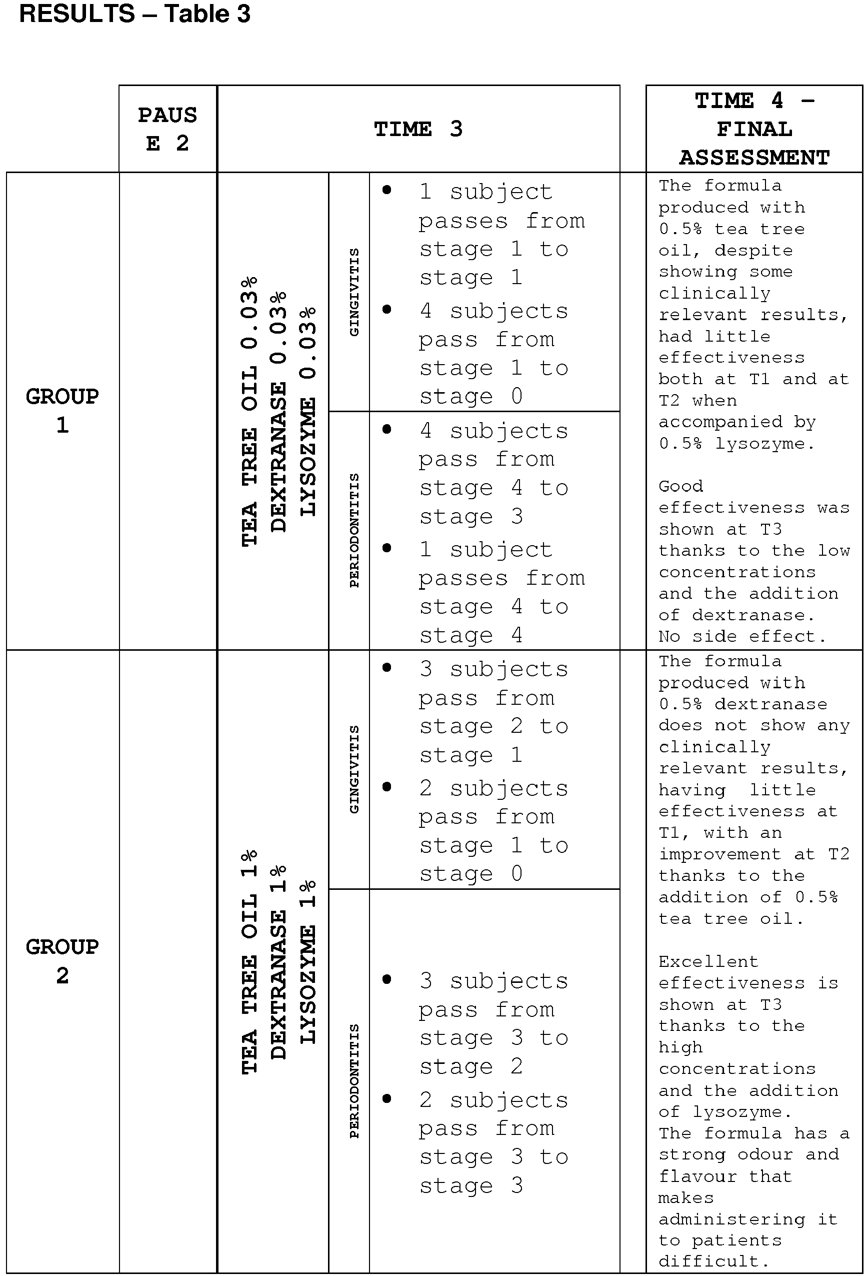

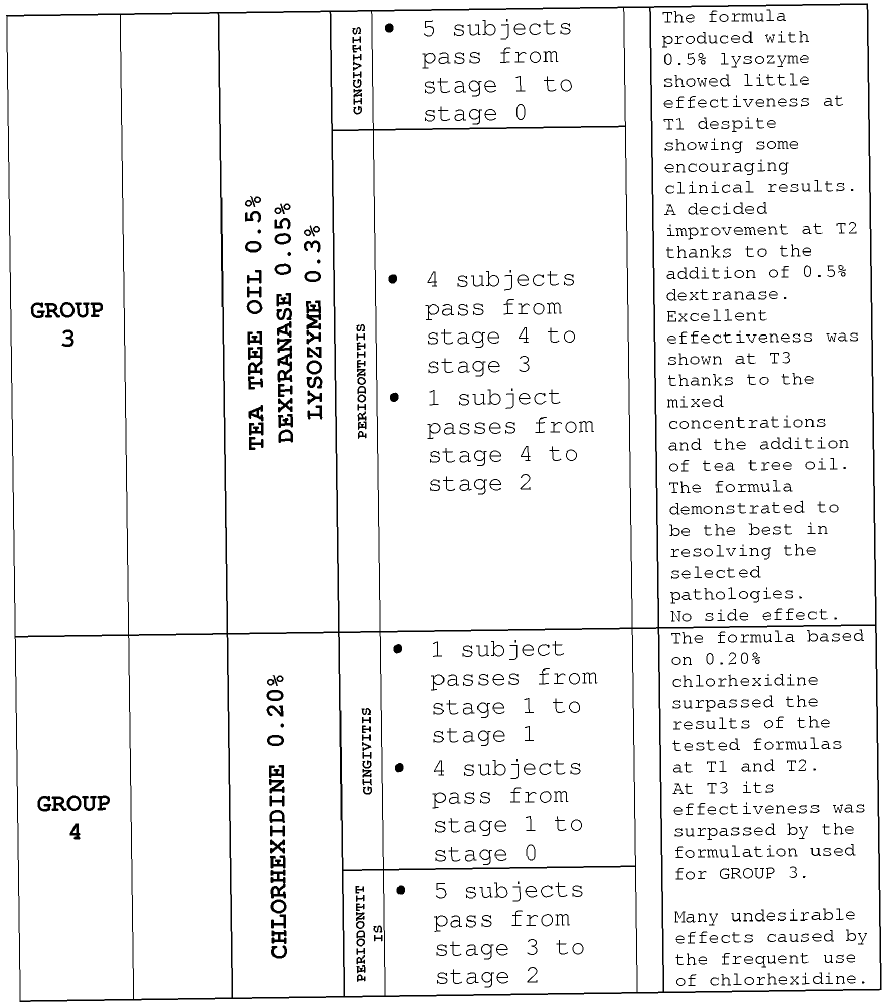

### EXAMPLE 4 - Pharmacological activity

**TREATMENT:** A group of 23 patients were selected in order to assess the pharmacological activity of a mouthwash containing tea tree oil, dextranase, lysozyme, extract of Centella asiatica, papain and idebenone delivered with natural carrageenan and xanthan gum.

The group of patients included 17 females and 6 males of an age comprised from 30 to 79 years, with a mean age of 60.7 and a standard deviation of 13.5 years. The majority of the pathologies of the oral cavity and the conditions affecting the group of patients were of an inflammatory and/or autoimmune type.

The pathologies tested were: oral lichen planus and the consequent desquamative gingivitis, migratory glossitis, xerostomia, xerostomia due to Sjögren's syndrome, burning mouth syndrome (BMS), oral candidiasis, benign mucous membrane pemphigoid (MMP), graft-versus-host disease (GvHD), prosthetic stomatitis, recurrent aphthosis and white tongue.

**RESULTS:** After a period of 15 days/1 month during which the patients did rinses of the oral cavity, the patients were called back and the oral pathologies were reassessed. Of the 23 patients, 3 did not show up for the appointment. Effects on the improvement of xerostomia, xerostomia due to Sjögren's syndrome, the taste of the mouthwash and soothing effects were found in 18 patients out of 23 (78.3%), whereas only 2 patients found no relief or palatability (8.7%). Of the 13 patients of the group (56.5%) affected by *oral lichen planus* and with vesicular-bullous lesions caused by pemphigoid, 8 patients had a visible improvement of the condition of the oral cavity and a considerably improved quality of life.

In particular, after 15 days of therapy consisting in a treatment with 1 rinse in the morning and 1 in the evening, the patients affected by benign mucous membrane pemphigoid showed a decided improvement of symptoms, with a recovery of vascularisation, excellent lubrication of the oral cavity and excellent soothing effects.

After 15 days of therapy consisting in 1 rinse in the morning and 1 in the evening, the patients affected by desquamative gingivitis due to lichen planus showed an excellent improvement of symptoms, excellent control of plaque formation and excellent lubrication of the oral cavity.

After 15 days of therapy consisting in 1 rinse in the morning and 1 in the evening, the patients affected by migratory glossitis showed a decided improvement of the clinical appearance and symptoms and an excellent lubrication of the oral cavity.

After 15 days of therapy consisting in 1 rinse in the morning and 1 in the evening, the patients affected by xerostomia and candidiasis showed an excellent improvement of symptoms, excellent control of plaque formation, disappearance of pigmentation and an improvement of the tongue coating. After 15 days of therapy consisting in 1 rinse in the morning and 1 in the evening, the patients affected by xerostomia and migratory glossitis showed a completely resolved migratory glossitis and an excellent improvement of the symptoms of xerostomia.

After 30 days of therapy consisting in 1 rinse in the morning and 1 in the evening, the patients affected by xerostomia due to Sjögren's syndrome showed an excellent improvement of the symptoms, excellent control of plaque formation and excellent lubrication of the oral cavity.

After 15 days of therapy consisting in 1 rinse in the morning and 1 in the evening, the patients affected by xerostomia, BMS and white tongue showed a good improvement of symptoms and excellent lubrication of the oral cavity.

After 30 days of therapy consisting in 1 rinse in the morning and 1 in the evening, the patients affected by graft-versus-host disease showed an excellent improvement of the clinical appearance and symptoms, excellent control of plaque in a patient with little motivation and excellent lubrication of the oral cavity.

After 15 days of therapy consisting in 1 rinse in the morning and 1 in the evening, the patients affected by prosthetic stomatitis and candidiasis showed an excellent clinical improvement and improvement of symptoms, excellent lubrication of the oral cavity and excellent soothing effects.

After 5 days of therapy consisting in 1 rinse in the morning and 1 in the evening, the patients affected by recurrent aphthosis showed an excellent improvement of symptoms, ulcers in the process of healing and excellent soothing effects.

After 30 days of therapy consisting in 1 rinse in the morning and 1 in the evening, the patients affected by oral lichen planus showed an excellent improvement of the symptoms, disappearance of the lesion and excellent soothing and lubrication effects.

**DISCUSSION AND CONCLUSIONS:** The mouthwash clearly showed a calming and soothing effect in patients complaining of xerostomia, xerostomia due to Sjögren's syndrome and BMS, and had a visible improvement and healing effect on the reticular lesions and blisters of patients affected by inflammatory and autoimmune diseases. This success is due to the synergistic effect of the active ingredients present in the mouthwash, which, despite being known individually for their antibacterial activity, unexpectedly demonstrated to be synergistically effective against inflammatory and autoimmune pathologies.

## Claims

1. A pharmaceutical composition comprising lysozyme, dextranase and an essential oil of a plant belonging to the family Myrtaceae for use as a medication.

2. The pharmaceutical composition for use according to claim 1, comprising a further ingredient selected in the group consisting of: papain, carrageenan, for example extract of Chondrus Crispus, xylitol, idebenone, extract of Centella asiatica, zeolite, bentonite, kaolin, ionic silver, colloidal silver, colloidal silica, colloidal copper, colloidal gold, casein phosphopeptide, amorphous calcium fluoride, glucose oxidase (GOX), lactoperoxidase, lactoferrin and mixtures thereof.

3. The composition for use according to claim 1 or 2, wherein the lysozyme is present in an amount ranging from 0.03% to 2% by weight, preferably from 0.1% to 0.5% by weight, more preferably from 0.2% to 0.4% by weight.

4. The composition for use according to any one of claims 1 to 3, wherein the dextranase is present in an amount comprised from 0.03% to 2% by weight, preferably from 0.05% to 0.3% by weight, more preferably from 0.05% to 0.1% by weight.

5. The composition for use according to any one of claims 1 to 4, wherein the essential oil is present in an amount comprised from 0.03% to 3% by weight, preferably from 0.3 to 0.8% by weight, more preferably from 0.4% to 0.6% by weight.

6. The composition for use according to any one of claims 1 to 5, wherein the essential oil of a plant belonging to the family Myrtaceae is selected from: essential oil of Melaleuca alternifolia (also called tea tree oil), essential oil of Kunzea ericoides (also called kanuka) and essential oil of leptospermum scoparium (also called manuka).

7. The composition for use according to any one of claims 1 to 6, formulated as a mouthwash, solution, suspension, gel, powder to be reconstituted in water, emulsion, oleolite, paste, foam, ointment, poultice, unguent, cream, lozenge, pill, capsule, tablet or chewing gum.

8. The composition for use according to any one of claims 1 to 7, in the prevention, treatment and follow-up of pathologies of the oral cavity of infectious (bacterial, fungal, viral), inflammatory, degenerative or autoimmune origin.

9. The composition for use according to claim 8, wherein said pathologies are selected in the group consisting of: gingivitis, periodontitis, aphthous stomatitis, prosthetic stomatitis, recurrent aphthosis, xerostomia, xerostomia due to Sjögren's syndrome, burning mouth syndrome (BMS), oral candidiasis, oral lichen planus, mucous membrane pemphigoid (MMP), graft-versus-host disease, white tongue, migratory glossitis, desquamative gingivitis due to oral lichen planus, herpes and combinations thereof.

10. A mouthwash for use according to any one of claims 1 to 9, comprising
• lysozyme in an amount of 0.03% to 2% by weight, preferably 0.1% to 0.5% by weight, more preferably 0.2% to 0.4% by weight;
• dextranase in an amount comprised from 0.03% to 2% by weight, preferably from 0.05% to 0.3% by weight, more preferably from 0.05% to 0.1% by weight;
• essential oil of a plant belonging to the family Myrtaceae in an amount of 0.03% to 3% by weight, preferably 0.3 to 0.8% by weight, more preferably 0.4% to 0.6% by weight;
• one or more further ingredients;
• water quantum sufficit.

11. The mouthwash for use according to claim 10, wherein said further ingredient is selected from: papain, xylitol, idebenone, extract of Centella asiatica, carrageenan and combinations thereof.

12. The mouthwash for use according to claim 10 or 11, wherein said essential oil of a plant belonging to the family Myrtaceae is essential oil of Melaleuca alternifolia (tea tree oil).

13. The mouthwash for use according to any one of claims 10 to 12, comprising sodium benzoate and acesulfame potassium.
